# EUROPEAN PATENT SPECIFICATION

(11) **EP 4 119 186 B1**
(45) Date of publication and mention of the grant of the patent: **30.07.2025**
(21) Application number: 20939168.9
(22) Date of filing: 06.11.2020
(51) Int. Cl.: A61N 1/375, F16B 2/06, F16B 35/04, A61N 1/05, F16B 19/00, H01R 13/52, H01R 13/621, H01R 13/639

(54) **ELECTRICAL NERVE STIMULATION SYSTEM**
ELEKTRISCHES NERVENSTIMULATIONSSYSTEM
SYSTEME DE STIMULATION ELECTRIQUE DES NERFS

(30) Priority: 02.06.2020 CN 202010487367
(43) Date of publication of application: 18.01.2023
(73) Proprietor: SceneRay Co., Ltd., Jiangsu 215123 (CN)
(72) Inventor: ZHU, Weiran, Suzhou, Jiangsu 215000 (CN); ZHANG, Haitao, Suzhou, Jiangsu 215000 (CN)
(74) Representative: Cabinet Beaumont
(86) International application number: PCT/CN2020/126903
(87) International publication number: WO 2021/243952

(56) References cited:
- EP-B1- 2 827 941
- CN-A- 103 893 911
- CN-A- 107 335 135
- CN-A- 110 732 083
- CN-A- 111 529 936
- CN-A- 111 529 937
- CN-U- 204 230 581
- CN-U- 210 114 745
- US-A1- 2012 016 452
- US-A1- 2017 100 596

## Description

### FIELD

This application relates to the technical field of medical devices, in particular to a neuro electrical stimulation system.

### BACKGROUND

Neuro electrical stimulation is a commonly used means for treatment of neuro dysfunction and neuro injury rehabilitation. Typically, a neuro electrical stimulation system includes a pulse generator, an extension wire and a lead implanted in the body, and a control apparatus located outside the body. One end of the extension wire is connected to the pulse generator and the other end is connected to the lead, so that pulses generated by the pulse generator are transmitted to the lead to electrically stimulate the neuro. Since the pulse generator, the extension wire and the lead are implanted into the human body, it must be ensured that the connection between the extension wire and the lead is tight, so as to avoid unnecessary operations during use. In the current neuro electrical stimulation system, the method for fixing the lead is generally that after connecting the lead to the extension wire, a sealing sleeve (silica gel protective sleeve) is added to the connector for sealing and fixing. However, since the sealing sleeve and the connector are of two separate parts, the assembly between the sealing sleeve and the connector is required to be considered, and the sealing sleeve finally has two ends thereof tied with strings to achieve the object of sealing. This type of sealing method has a low reliability and poor stability. In addition, when the extension wire corresponds to a conductive ring of the lead at the joint and is connected, the conductive ring is required to be locked by screws to realize the electrical connection between the lead and the extension wire, but this method damages the conductive ring and adversely affects the electrical connection effect.

Further relevant technologies are also known from CN 107 335 135 A which relates to electrode socket sleeve and electrode socket assembly, EP 2 827 941 B1 which relates to torque lock anchor and methods and devices using the anchor and US 2012/0016452 A1 which relates to high-resolution connector for a neurostimulation lead.

### SUMMARY

An aspect of the present application is to provide a neuro electrical stimulation system with a simple fixing means for fixing a lead to an extension wire, and the fixing means has a simple structure, is simple to manufacture, and has a good sealing performance, a low cost and a high reliability.

The invention is set out in the appended set of claims.

### BRIEF DESCRIPTION OF DRAWINGS

FIG. 1 is a schematic structural diagram of an extension wire with a connector according to an embodiment of the present application;
FIG. 2 is a cross-sectional view of the extension wire with the connector shown in FIG. 1;
FIG. 3 is a schematic structural view of a fixing assembly with a sealing block in FIG. 1;
FIG. 4 is a schematic structural view of a fixing block in FIG. 1;
FIG. 5 is a schematic structural view of a fixing member in FIG. 1; and
FIG. 6 is a cross-sectional view of the fixing member in FIG. 1.

### DETAILED DESCRIPTION

The embodiments of the present application are described in further detail hereinafter with reference to the drawings and examples. The following examples are intended to illustrate the present application, rather than limiting the scope of the present application.

In order to treat diseases such as neuro dysfunction and neuro damage in the human body, the neuro electrical stimulation system shown in the present application is required to co-implant a pulse generator, an extension wire connected to the pulse generator, and a lead connected to the extension wire into a human body, and an electrode in the lead is used to electrically stimulate a neuro. The extension wire is provided with a channel therein, and the lead is inserted into the channel and is abutted with the extension wire. In order to increase stability of the connection between the extension wire and the lead, a fixing assembly is arranged in the channel to restrict the movement of the lead in the channel.

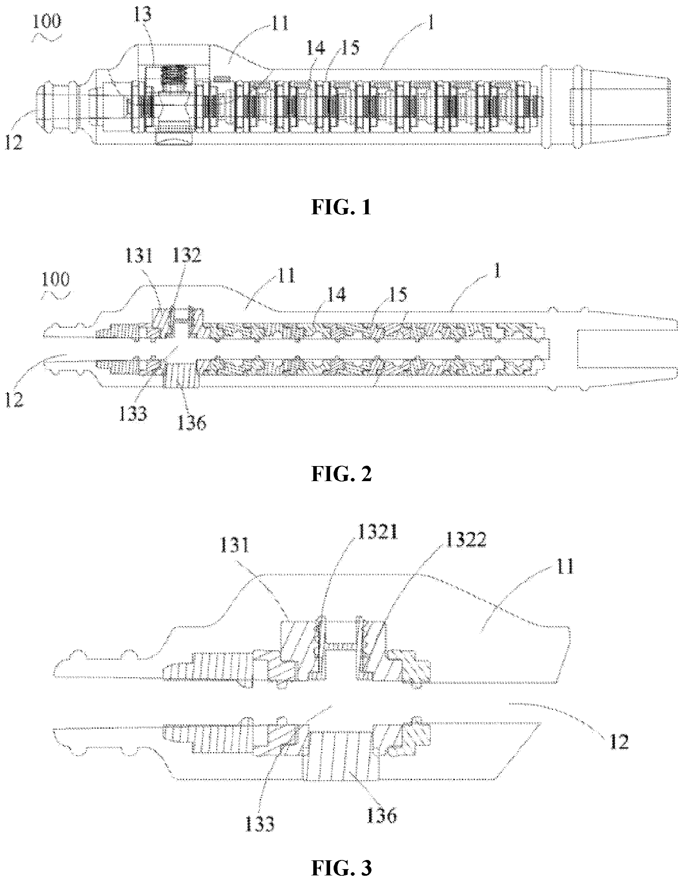

Referring to FIG. 1 and FIG. 2, one end of an extension wire 100 shown in an embodiment of the present application is provided with a connector 1 for connecting with a lead (not shown), the connector 1 includes a housing 11, and the housing 11 is formed of silica gel, and the housing 11 is formed therein with a channel 12 in which the lead can be inserted and a fixing assembly 13 for fixing the lead. The silica gel coats the fixing assembly 13 to obtain the connector 1 with a good sealing performance.

The housing 11 is further provided therein with a number of connecting blocks 14 for electrical connection with the lead. In this embodiment, the number of the connecting blocks 14 is eight. In other embodiments, the number of the connecting blocks can be set on the basis of practical requirements, which is not specifically limited here. The connecting blocks 14 are arranged at equal distances, and an insulating member 15 is arranged between every two connecting blocks 14 adjacent to each other, and the insulating member 15 is made of an insulating material, and the insulating material is not specifically limited herein. The connecting blocks 14 and the insulating members 15 are arranged in the housing 11, and each of the connecting blocks 14 and the insulating members 15 has a connecting hole (not shown), and the connecting hole is coaxial with the channel 12, two ends of the connecting hole are each in communication with the channel 12, and it may also be understood as that the connecting hole constitutes a part of the channel 12. The connecting block 14 is electrically connected to a conductive wire (not shown) in the extension wire 100, and the connecting block 14 is also electrically connected to a conductive ring of the lead inserted in this position, so as to realize the electrical connection between the lead and the extension wire; where the conductive wire can be welded to the connecting block 14, and the connecting block 14 can be connected to the conductive ring of the lead by a conductive spring (not shown); or the conductive wire is electrically connected to a pin of a conductive spring extending from the connecting block 14. In other embodiments, the electrical connection between the connecting block and the lead may also be implemented in other manners, which are not limited to the conductive spring, and are not listed one by one here.

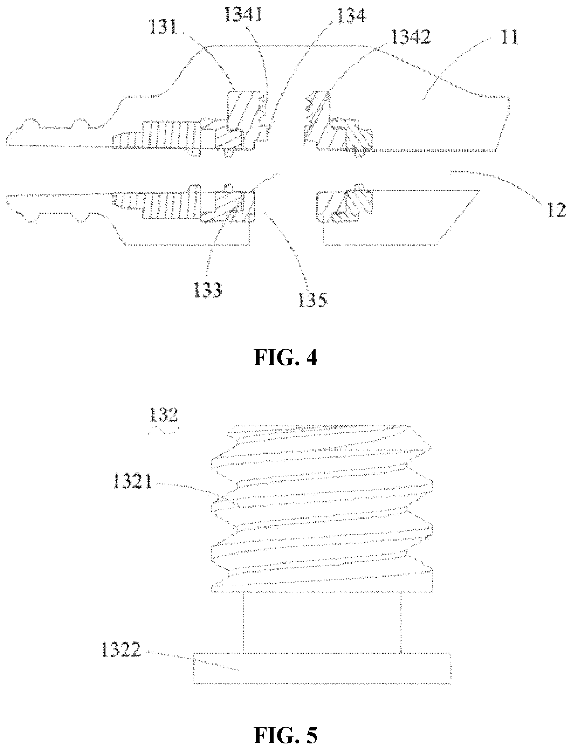

Referring to FIG. 3 and FIG. 4, the fixing assembly 13 is arranged on one side of the connecting block 14, and the fixing assembly 13 includes a fixing block 131 which is at least partially embedded in the channel 12, and a fixing member 132 which can be locked into the fixing block 131. The fixing block 131 is provided with a receiving hole 133, and the receiving hole 133 penetrate through the he fixing block 131 and is used for the lead to pass through. The receiving hole 133 is arranged to be coaxial with the channel 12 and two ends of the receiving hole 133 are each in communication with the channel 12. Similarly, it may also be understood as that the receiving hole 133 constitutes a part of the channel 12. The fixing block 131 is provided with a fixing hole 134 in communication with the receiving hole 133. The axis in which the fixing hole 134 is located is perpendicular to the axis in which the receiving hole 133 is located. Specifically, the fixing hole 134 is arranged above the receiving hole 133. It is true that in other embodiments, the axis in which the fixing hole 134 is located and the axis in which the receiving hole 133 is located may intersect, and an included angle between the two axes is greater than zero.

The fixing member 132 is at least partially inserted into the receiving hole 133 through the fixing hole 134. The fixing member 132 is inserted into the fixing block 131 and then clamps the lead with the fixing block 131, thereby fixing the lead into the channel 12. Referring to FIG. 5, the fixing member 132 includes a locking portion 1321 and a protruding portion 1322, the locking portion 1321 is fitted with the fixing block 131 to realize the locking of the fixing member 132 to the fixing block 131, and the protruding portion 1322 protrudes outward from one end of the locking portion 1321. The fixing member 132 is a substantially T-shaped structure, and the locking portion 1321 and the protruding portion 1322 are integrally formed, the locking portion 1321 is a cylindrical structure, and its outer circumference has a threaded portion that can form a thread fit, and the threaded portion is formed on a part of a circumferential surface of the locking portion 1321. In other embodiments, the threaded portion may also be formed on the entire circumferential surface of the locking portion 1321. The structure of the protruding portion 1322 can be understood as an annular structure and being sleeved on the outer circumference of one end of the locking portion 1321. Therefore, the diameter of the protruding portion 1322 is larger than the diameter of the locking portion 1321. The fixing member 132 may be a screw having the same structure as the structure described above, but is not limited thereto.

The fixing hole 134 in the fixing block 131 includes a locking hole 1341 for the locking portion 1321 to be inserted therein and locked, and a receiving cavity 1342 for receiving the protruding portion 1322. The locking hole 1341 has a threaded portion that can be threadedly fitted with the locking portion 1321. The receiving cavity 1342 is located below the locking hole 1341 and is in communication with the receiving hole 133. The protruding portion 1322 can be at least partially located in the receiving hole 133 and can move in the receiving hole 133. That the fixing member 132 and the fixing block 131 clamp the lead specifically be clamping the lead through the protruding portion 1322 and the fixing block 131.

The fixing block 131 is further provided with a mounting hole 135 that is in communication with the receiving hole 133. The mounting hole 135 and the fixing hole 134 are co-axially and oppositely arranged on two sides of the receiving hole 133. The cross-section of the mounting hole 135 is a convex structure, and the axis in which the mounting hole 135 is located intersects with the axis in which the receiving hole 133 is located. In this embodiment, the axis in which the mounting hole 135 is located is perpendicular to the axis in which the receiving hole 133 is located. The diameter of the mounting hole 135 is not smaller than the diameter of the protruding portion 1322, that is, the diameter of the mounting hole 135 is not smaller than the diameter of the receiving cavity 1342, so as to ensure that the fixing member 132 can be moved into the fixing hole 134 through the mounting hole 135 and locked to the fixing hole 134. The mounting hole 135 may further be provided with a sealing block 136 therein for sealing the mounting hole 135. The shape of the sealing block 136 matches the shape of the mounting hole 135. The sealing block 136 is inserted into the mounting hole 135 to block and seal the mounting hole 135.

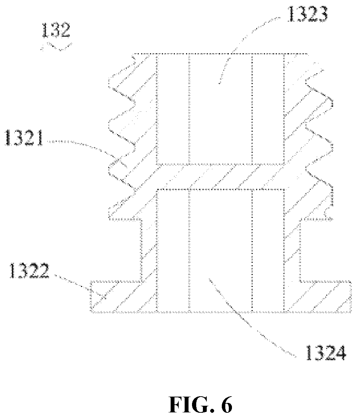

Referring to FIG. 6, the fixing member 132 is provided with a force-applying portion for applying an external force to move the fixing member 132 relative to the receiving hole 133. The force-applying portion includes a first groove 1323 and a second groove 1324, the first groove 1323 is concavely formed from a surface of the locking portion 1321 of the fixing member 132 and can be fitted with a locking tool (for example, a screw knife, an inner hexagon wrench), and the second groove 1324 is concavely formed from a surface of the protruding portion 1322 of the fixing member 132 and can be fitted with the locking tool. The first groove 1323 and the second groove 1324 can be a hexagonal groove or a cross-shaped groove, and it is easy to be conceived that the force applying portion can also be a structure that can be fitted with other tools.

In this embodiment, the manufacturing process of the connector 1 is as follows: the connecting blocks 14, the insulating members 15 and the fixing block 131 are arranged at equal distances to form the channel 12, as shown in FIG. 1 and FIG. 2, the fixing hole 134 and the mounting hole 135 in the fixing block 131 are sealed with PIN pins, the insulating members 15 are arranged between the connecting blocks 14 or between the fixing assembly 13 and the connecting blocks 14, and a silica gel is coated on outer sides of the connecting blocks 14 and the fixing assembly 13 to form the connector 1 having the channel 12.

The method for fixing the extension wire 100 to the lead is as follows: first, the PIN pins located in the fixing hole 134 and the mounting hole 135 respectively are taken out, and a portion of the silicone gel above the positions of the fixing hole 134 and the mounting hole 135 is cut to have a slot. The substantially T-shaped fixing member 132, after being upside down, is disposed into the fixing block 131 from the mounting hole 135. A screwdriver or a wrench is used to protrude into the first groove 1323 and rotate the fixing member 132, and the threaded portion on the outer circumference of the locking portion 1321 is fitted with the threaded portion in the fixing hole 134, so that the fixing member 132 enters the fixing block 131 and locked to the fixing block 131. At this time, the locking portion 1321 enters the fixing hole 134, and the protruding portion 1322 enters the receiving cavity 1342 via the mounting hole 135 and the receiving hole 133, and finally, the sealing block 136 is inserted into the mounting hole 135 to seal the mounting hole 135. Then, the lead is inserted into the channel 12 of the connector 1, passes through the receiving hole 133 and reaches the desired position, to allow the lead to be electrically connected to the extension wire 100. A screwdriver or a wrench is used to pass through the slot to screw the locking portion 1321 downward, where the torque wrench or screwdriver acts on the locking portion 1321 and is inserted into the second groove 1324. The threaded portion on the outer circumference of the locking portion 1321 is fitted with the threaded portion in the fixing hole 134, so that the protruding portion 1322 is inserted into the receiving hole 133 and contacts with the extension wire 100. The locking portion 1321 is continued to be screwed downward, so that finally the protruding portion 1322 presses the extension wire 100 tightly into the channel 12, and the lead and the extension wire 100 are fixed relative to each other, and after finishing, the torque wrench or screwdriver is then taken out, and the opened slot is automatically closed to achieve the sealing effect. In the method, the sealing sleeve outside the connector is removed, which, compared with the method in which the two ends of the sealing sleeve must be fastened with strings after the extension wire and the lead are connected, has a better reliability and a simpler structure, significantly reduces effort and manufacturing time, while reducing costs.

To sum up, the extension wire of the neuro electrical stimulation system involved in the present application has a channel, the lead is inserted into the channel and is abutted with the extension wire, and the channel is provided therein with a fixing assembly for restricting the movement of the lead in the channel. In the method, the step of being additionally sealed after the lead is abutted with the extension wire is exempted, and the used fixing assembly has a simple structure, is simple to manufacture, has a good sealing performance, low cost, is time-saving, labor-saving and reliable.

The technical features of the above embodiments can be combined arbitrarily. For the sake of brevity, not all possible combinations of the technical features in the above embodiments are described. However, as long as there is no contradiction in the combinations of these technical features, those combinations should be considered as falling into the range set forth in this specification.

## Claims

1. A neuro electrical stimulation system, comprising an extension wire (100) and a lead connected to the extension wire (100), wherein the extension wire (100) is provided with a channel (12), and the lead is inserted into the channel (12) and abutted with the extension wire (100), a fixing assembly (13) is arranged in the channel (12) and configured to restrict the lead to move in the channel (12);
wherein the fixing assembly (13) comprises a fixing block (131) at least partially embedded in the channel (12), and a fixing member (132) capable of being locked into the fixing block (131), the fixing block (131) is provided with a receiving hole (133), the receiving hole (133) penetrates through the fixing block (131) and is configured for the lead to pass through, two ends of the receiving hole (133) are each in communication with the channel (12), the fixing block (131) is provided with a fixing hole (134) that is in communication with the receiving hole (133), the fixing member (132) is at least partially inserted into the receiving hole (133) through the fixing hole (134), and configured to, after said fixing member (132) is inserted into the fixing block (131), clamp the lead with the fixing block (131);
wherein the fixing member (132) comprises a locking portion (1321) and a protruding portion (1322), the locking portion (1321) is fitted with the fixing block (131) to realize locking between the fixing member (132) and the fixing block (131);
wherein the neuro electrical stimulation system is **characterized in that**, the protruding portion (1322) is formed by protruding outwards from one end of the locking portion (1321), the protruding portion (1322) is at least partially located in the receiving hole (133) and is movable in the receiving hole (133), and the fixing member (132) is configured to clamp the lead with the fixing block (131) through the protruding portion (1322), the locking portion (1321) and the protruding portion (1322) are integrally formed, the fixing hole (134) includes a locking hole (1341) for the locking portion (1321) to be inserted therein and locked, and a receiving cavity (1342) for receiving the protruding portion (1322);
wherein the fixing block (131) is further provided with a mounting hole (135) that is in communication with the receiving hole (133), the mounting hole (135) and the fixing hole (134) are coaxially and oppositely arranged on two sides of the receiving hole (133), the mounting hole (135) has a diameter not smaller than a diameter of the protruding portion (1322), and an axis in which the mounting hole (135) is located intersects with an axis in which the receiving hole (133) is located; the fixing member (132) is configured to be moved into the fixing hole (134) through the mounting hole (135) and to be locked to the fixing hole (134).

2. The neuro electrical stimulation system according to claim 1, wherein the receiving hole (133) is arranged coaxially with the channel (12).

3. The neuro electrical stimulation system according to claim 1, wherein an axis in which the fixing hole (134) is located is perpendicular to an axis in which the receiving hole (133) is located.

4. The neuro electrical stimulation system according to claim 1, wherein a sealing block (136) is provided in the mounting hole (135) to seal the mounting hole (135).

5. The neuro electrical stimulation system according to claim 1, wherein the extension wire (100) comprises a housing (11), the channel (12) is provided in the housing (11), and the housing (11) is made of silica gel and coats the fixing assembly (13).

6. The neuro electrical stimulation system according to claim 1, wherein the fixing member (132) is provided with a force-applying portion configured to apply an external force to drive the fixing member (132) to move relative to the receiving hole (133), and the force-applying portion comprises: a first groove (1323) and a second groove (1324), wherein the first groove (1323) is formed concavely from a surface of the locking portion (1321) of the fixing member (132) and capable of being fitted with a locking tool, and the second groove (1324) is formed concavely from a surface of the protruding portion (1322) of the fixing member (132) and capable of being fitted with a locking tool.

## Patentansprüche

1. Neuro-Elektrostimulationssystem, das einen Verlängerungsdraht (100) und eine mit dem Verlängerungsdraht (100) verbundene Leitung umfasst, wobei der Verlängerungsdraht (100) mit einem Kanal (12) versehen ist und die Leitung in den Kanal (12) eingeführt wird und an den Verlängerungsdraht (100) anstößt, wobei eine Befestigungsanordnung (13) in dem Kanal (12) angeordnet und so konfiguriert ist, dass sie die Bewegung der Leitung in dem Kanal (12) einschränkt;
wobei die Befestigungsanordnung (13) einen Befestigungsblock (131), der zumindest teilweise in den Kanal (12) eingebettet ist, und ein Befestigungselement (132) umfasst, das in den Befestigungsblock (131) verriegelt werden kann, wobei der Befestigungsblock (131) mit einem Aufnahmeloch (133) versehen ist, das durch den Befestigungsblock (131) hindurchgeht und so konfiguriert ist, dass die Leitung hindurchgehen kann, wobei zwei Enden des Aufnahmelochs (133) jeweils mit dem Kanal (12) in Verbindung stehen, wobei der Befestigungsblock (131) mit einem Befestigungsloch (134) versehen ist, das mit dem Aufnahmeloch (133) in Verbindung steht, wobei das Befestigungselement (132) zumindest teilweise durch das Befestigungsloch (134) in dem Aufnahmeloch (133) eingeführt ist und so konfiguriert ist, dass es die Leitung mit dem Befestigungsblock (131) festklemmt, nachdem das Befestigungselement (132) in dem Befestigungsblock (131) eingeführt ist;
wobei das Befestigungselement (132) einen Verriegelungsabschnitt (1321) und einen vorstehenden Abschnitt (1322) umfasst, wobei der Verriegelungsabschnitt (1321) mit dem Befestigungsblock (131) zusammengefügt ist, um eine Verriegelung zwischen dem Befestigungselement (132) und dem Befestigungsblock (131) zu realisieren;
wobei das Neuro-Elektrostimulationssystem **dadurch gekennzeichnet ist, dass** der vorstehende Abschnitt (1322) dadurch gebildet wird, dass er von einem Ende des Verriegelungsabschnitts (1321) nach außen vorsteht, wobei der vorstehende Abschnitt (1322) zumindest teilweise in dem Aufnahmeloch (133) angeordnet ist und in dem Aufnahmeloch (133) beweglich ist, wobei das Befestigungselement (132) so konfiguriert ist, dass es die Leitung mit dem Befestigungsblock (131) durch den vorstehenden Abschnitt (1322) festklemmt, wobei der Verriegelungsabschnitt (1321) und der vorstehende Abschnitt (1322) einstückig ausgebildet sind, wobei das Befestigungsloch (134) ein Verriegelungsloch (1341), in das der Verriegelungsabschnitt (1321) einzuführen und zu verriegeln ist, und einen Aufnahmehohlraum (1342) zur Aufnahme des vorstehenden Abschnitts (1322) aufweist;
wobei der Befestigungsblock (131) ferner mit einem Montageloch (135) versehen ist, das mit dem Aufnahmeloch (133) in Verbindung steht, wobei das Montageloch (135) und das Befestigungsloch (134) koaxial und gegenüberliegend auf zwei Seiten des Aufnahmelochs (133) angeordnet sind, wobei das Montageloch (135) einen Durchmesser aufweist, der nicht kleiner ist als ein Durchmesser des vorstehenden Abschnitts (1322), und wobei eine Achse, in der das Montageloch (135) angeordnet ist, sich mit einer Achse schneidet, in der das Aufnahmeloch (133) angeordnet ist; wobei das Befestigungselement (132) so konfiguriert ist, dass es durch das Montageloch (135) in das Befestigungsloch (134) bewegt und mit dem Befestigungsloch (134) verriegelt werden kann.

2. Neuro-Elektrostimulationssystem nach Anspruch 1, wobei das Aufnahmeloch (133) koaxial zum Kanal (12) angeordnet ist.

3. Neuro-Elektrostimulationssystem nach Anspruch 1, wobei eine Achse, in der das Befestigungsloch (134) angeordnet ist, senkrecht zu einer Achse verläuft, in der das Aufnahmeloch (133) angeordnet ist.

4. Neuro-Elektrostimulationssystem nach Anspruch 1, wobei ein Dichtungsblock (136) in dem Montageloch (135) vorgesehen ist, um das Montageloch (135) abzudichten.

5. Neuro-Elektrostimulationssystem nach Anspruch 1, wobei der Verlängerungsdraht (100) ein Gehäuse (11) umfasst, in dem der Kanal (12) vorgesehen ist, und wobei das Gehäuse (11) aus Kieselgel besteht und die Befestigungsanordnung (13) umhüllt.

6. Neuro-Elektrostimulationssystem nach Anspruch 1, wobei das Befestigungselement (132) mit einem Kraftaufbringungsabschnitt versehen ist, der so konfiguriert ist, dass er eine äußere Kraft aufbringt, um das Befestigungselement (132) anzutreiben, sich relativ zu dem Aufnahmeloch (133) zu bewegen, wobei der Kraftaufbringungsabschnitt eine erste Nut (1323) und eine zweite Nut (1324) umfasst, wobei die erste Nut (1323) ausgehend von einer Oberfläche des Verriegelungsabschnitts (1321) des Befestigungselements (132) konkav ausgebildet ist und mit einem Verriegelungswerkzeug zusammenwirken kann, und wobei die zweite Nut (1324) ausgehend von einer Oberfläche des vorstehenden Abschnitts (1322) des Befestigungselements (132) konkav ausgebildet ist und mit einem Verriegelungswerkzeug zusammenwirken kann.

## Revendications

1. Système de neurostimulation électrique, comprenant un fil d'extension (100) et un fil connecté au fil d'extension (100), dans lequel le fil d'extension (100) est pourvu d'un canal (12), et le fil est inséré dans le canal (12) et est abouté au fil d'extension (100), et un ensemble de fixation (13) est disposé dans le canal (12) et configuré pour restreindre le fil à se déplacer dans le canal (12) ;
dans lequel l'ensemble de fixation (13) comprend un bloc de fixation (131) au moins partiellement encastré dans le canal (12), et un élément de fixation (132) capable d'être verrouillé dans le bloc de fixation (131), le bloc de fixation (131) est pourvu d'un trou de réception (133), le trou de réception (133) pénètre à travers le bloc de fixation (131) et est configuré pour permettre au fil de passer à travers celui-ci, chacune de deux extrémités du trou de réception (133) est en communication avec le canal (12), le bloc de fixation (131) est pourvu d'un trou de fixation (134) qui est en communication avec le trou de réception (133), l'élément de fixation (132) est au moins partiellement inséré dans le trou de réception (133) à travers le trou de fixation (134), et configuré pour, une fois ledit élément de fixation (132) inséré dans le bloc de fixation (131), serrer le fil avec le bloc de fixation (131) ;
dans lequel l'élément de fixation (132) comprend une portion de verrouillage (1321) et une portion saillante (1322), la portion de verrouillage (1321) est pourvue du bloc de fixation (131) pour réaliser un verrouillage entre l'élément de fixation (132) et le bloc de fixation (131) ;
dans lequel le système de neurostimulation électrique est **caractérisé en ce que**, la portion saillante (1322) est formée en faisant saillie vers l'extérieur à partir d'une extrémité de la portion de verrouillage (1321), la portion saillante (1322) est au moins partiellement située dans le trou de réception (133) et est mobile dans le trou de réception (133), et l'élément de fixation (132) est configuré pour serrer le fil avec le bloc de fixation (131) à travers la portion saillante (1322), la portion de verrouillage (1321) et la portion saillante (1322) sont formées intégralement, le trou de fixation (134) comprend un trou de verrouillage (1341) pour permettre à la portion de verrouillage (1321) d'être insérée dans celui-ci et verrouillée, et une cavité de réception (1342) pour recevoir la portion saillante (1322) ;
dans lequel le bloc de fixation (131) est pourvu en outre d'un trou de montage (135) qui est en communication avec le trou de réception (133), le trou de montage (135) et le trou de fixation (134) sont disposés de manière coaxiale et sont opposée l'un de l'autre sur deux côtés du trou de réception (133), le trou de montage (135) a un diamètre non inférieur à un diamètre de la portion saillante (1322), et un axe où se trouve le trou de montage (135) coupe un axe où se trouve le trou de réception (133) ; l'élément de fixation (132) est configuré pour se déplacer jusqu'à l'intérieur du trou de fixation (134) à travers le trou de montage (135) et pour être verrouillé sur le trou de fixation (134).

2. Système de neurostimulation électrique selon la revendication 1, dans lequel le trou de réception (133) est disposé coaxialement avec le canal (12).

3. Système de neurostimulation électrique selon la revendication 1, dans lequel un axe où se trouve le trou de fixation (134) est perpendiculaire à un axe où se trouve le trou de réception (133).

4. Système de neurostimulation électrique selon la revendication 1, dans lequel un bloc d'étanchéité (136) est prévu dans le trou de montage (135) pour fermer le trou de montage (135).

5. Système de neurostimulation électrique selon la revendication 1, dans lequel le fil d'extension (100) comprend un boîtier (11), le canal (12) est prévu dans le boîtier (11) et le boîtier (11) est réalisé en gel de silice et recouvre l'ensemble de fixation (13).

6. Système de neurostimulation électrique selon la revendication 1, dans lequel l'élément de fixation (132) est pourvu d'une portion d'application de force configurée pour appliquer une force externe pour entraîner l'élément de fixation (132) à se déplacer par rapport au trou de réception (133), et la portion d'application de force comprend : une première rainure (1323) et une seconde rainure (1324), dans laquelle la première rainure (1323) est formée de manière concave à partir d'une surface de la portion de verrouillage (1321) de l'élément de fixation (132) et capable d'être pourvue d'un outil de verrouillage, et la seconde rainure (1324) est formée de manière concave à partir d'une surface de la portion saillante (1322) de l'élément de fixation (132) et capable d'être pourvue d'un outil de verrouillage.
